(19) Europäisches Patentamt / European Patent Office / Office européen des brevets

(11) **EP 3 789 004 A1**

(12) **EUROPEAN PATENT APPLICATION**
published in accordance with Art. 153(4) EPC

(43) Date of publication:
**10.03.2021 Bulletin 2021/10**

(21) Application number: **18938925.7**

(22) Date of filing: **21.11.2018**

(51) Int Cl.:
*A61K 8/02* (2006.01)          *A61K 8/891* (2006.01)
*A61Q 1/02* (2006.01)          *C08G 18/40* (2006.01)

(86) International application number:
**PCT/KR2018/014364**

(87) International publication number:
**WO 2020/091132 (07.05.2020 Gazette 2020/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.10.2018 KR 20180130645**

(71) Applicant: **Cosmax, Inc.**
**Hwaseong-si, Gyeonggi-do 18622 (KR)**

(72) Inventors:
• **OH, Se Min**
**Suwon-si, Gyeonggi-do 16553 (KR)**

• **PARK, Sung Mi**
**Hwaseong-si, Gyeonggi-do 18482 (KR)**
• **LEE, Hwa Young**
**Yongin-si, Gyeonggi-do 16903 (KR)**
• **NOH, Min Joo**
**Seongnam-si, Gyeonggi-do 13628 (KR)**
• **LIM, Hyung Taek**
**Seoul 06289 (KR)**
• **JEON, Yong Seok**
**Seongnam-si, Gyeonggi-do 13586 (KR)**
• **PARK, Myeong Sam**
**Seoul 04987 (KR)**

(74) Representative: **Mammel und Maser**
**Patentanwälte**
**Tilsiter Straße 3**
**71065 Sindelfingen (DE)**

(54) **ETHER/ESTER COMPOSITE POLYURETHANE FOAM COSMETIC**

(57)     The present invention relates to a cosmetic comprising polyurethane foam in which a liquid cosmetic substance is impregnated. The present invention provides a cosmetic comprising polyurethane foam in which a cosmetic composition is impregnated, wherein the polyurethane of the polyurethane foam comprises an ether group and an ester group in a molar ratio of 4:6 to 6:4, the cosmetic composition has a water content of 35 wt% or less, and the threshold shear stress at which storage modulus (G') = loss modulus (G") is 100 Pa or less. According to the present invention, provided is a cosmetic comprising a composite polyurethane foam, wherein a cosmetic composition is stably present in a supported state and can be uniformly rearranged inside the foam during pressurization.

FIG. 4

**Description**

**Technical field**

The present invention relates to a cosmetic. More specifically, the present invention relates to a cosmetic comprising polyurethane foam in which a liquid cosmetic substance is impregnated.

**Background art**

A cosmetic substance exists in various forms such as solid, liquid, etc. In general, liquid cosmetic substances are used being stored in a container with elasticity such as a tube in order to prevent leakage and facilitate discharge.

Recently, cushion-type cosmetics are used by having a liquid cosmetic substance with low viscosity or high viscosity impregnated in an impregnating material such as polyurethane foam, and pressurizing the polyurethane foam using a tool such as a puff to discharge the cosmetic substance by soaking the cosmetic substance onto the puff.

Conventionally, polyurethane foam was mainly used as an impregnating material for such cosmetics. Such polyurethane foam may be classified into ether-based polyurethane foam and ester-based polyurethane foam according to the type of polyol used for the polymer polymerization. Ether-based polyurethane foam has excellent hydrolysis resistance, excellent elasticity and costs less, but has weak oil resistance. In comparison, ester-based polyurethane foam can adjust air bubbles easily, and has good abrasion resistance, chemical resistance, etc., but may cause hydrolysis easily. Accordingly, there is a demand for a polyurethane foam which may supplement the disadvantages and highlight the advantages of the two polyols.

In addition, as for the conventional cushion-type cosmetics, there was no contemplation on the material or structure of the foam, or any requirements for a cosmetic composition suitable therefor. No cosmetic composition has been designed based on such contemplation.

**Detailed description of invention**

**Technical task**

In order to solve the above problems of prior art, it is an object of the present invention to provide a cosmetic comprising an ester/ether composite polyurethane foam having a specific ratio of ether/ester.

It is another object of the present invention to provide a polyurethane foam cosmetic comprising a cosmetic composition having flow properties suitable for pressurized use.

More specifically, it is an object of the present invention to provide an ether/ester polyurethane foam cosmetic having chemical resistance and flow properties suitable for pressurized use.

**Means for solving technical task**

In order to achieve the above technical tasks, the present invention provides a cosmetic comprising polyurethane foam in which a cosmetic composition is impregnated, wherein the polyurethane of the polyurethane foam comprises an ether group and an ester group in a molar ratio of 4:6 to 6:4, the cosmetic composition has a water content of 35 wt% or less, and the threshold shear stress at which storage modulus (G')=loss modulus (G") is 100 Pa or less.

In the present invention, the water content of the cosmetic composition may be 20 to 35 wt%. Also, the water content of the cosmetic composition may be 20 to 30 wt%. Further, the water content of the cosmetic composition may be 25 to 30 wt%.

In the present invention, the threshold shear stress of the cosmetic composition may be 20 Pa or less, 10 Pa or less, or 5 Pa or less.

In the present invention, the cosmetic composition may be $3 \leq G'G" \leq 10$ under a shear stress of 1 Pa.

In the present invention, preferably, the cosmetic composition comprises an oil phase component and an aqueous phase component, and the weight ratio of the oil phase component to the aqueous phase component is in a range of 0.9 to 1.1.

Preferably, the cosmetic composition comprises a main emulsifying agent and an auxiliary emulsifying agent, and the weight ratio of the main emulsifying agent and the auxiliary emulsifying agent is in a range of 3:1 to 4:1.

At this time, preferably, the main emulsifying agent comprises at least one substance selected from the group consisting of lauryl PEG-10 tris (trimethylsiloxy) silylethyl dimethicone, lauryl PEG-9 polydimethyl siloxyethyl dimethicone and cetyl PEG/PPG-10/1 dimethicone, and the auxiliary emulsifying agent comprises at least one of PEG-10 dimethicone and sorbitan isostearate.

In the present invention, preferably, the hardness after impregnating the polyurethane foam is 20 to 35. At this time, more preferably, the hardness change rate before and after impregnation with respect to the hardness of the polyurethane foam before impregnation is 45% or less.

**Effect of invention**

According to the present invention, a cosmetic comprising an ester/ether composite polyurethane foam having a specific ratio of ether/ester may be provided.

Also, according to the present invention, a cosmetic comprising a composite polyurethane foam wherein a cosmetic composition is stably present in a supported state and can be uniformly rearranged inside the foam during pressurization may be provided.

**Brief description of drawings**

Fig. 1 is a schematic drawing of the cosmetic according to an embodiment of the present invention;

Fig. 2 is a two-dimensional conceptual drawing of the structure of the impregnating material of the present invention;

Figs. 3A and 3B are schematic drawings for illustrating the behavior of the impregnating material and the cosmetic composition in the present invention;

Fig. 4 is a schematic drawing for illustrating the flow of the cosmetic composition according to an embodiment of the present invention;

Fig. 5 is a graph illustrating the flow properties of the cosmetic composition according to an embodiment of the present invention;

Fig. 6 is a graph illustrating the flow properties of the cosmetic composition according to another embodiment of the present invention;

Fig. 7 is a graph illustrating the flow properties of the cosmetic composition according to yet another embodiment of the present invention; and

Figs. 8A and 8B are graphs illustrating the results of measuring the hardness before and after impregnation of the polyurethane foam prepared according to an embodiment of the present invention.

**Best mode for carrying out the invention**

Hereinafter, preferred embodiments according to the present invention will be described in detail with reference to the accompanying drawings.

In the specification of the present invention, the ether polyurethane foam comprises those processing a polyurethane formed by reacting polyether polyol with a compound having an isocyanate (-NCO) group as a foamed foam. In addition, the ester polyurethane foam comprises those processing a polyurethane formed by reacting polyester polyol with a compound having an isocyanate (-NCO) group as a foamed foam. Also, in the present invention, the ether/ester polyurethane foam comprises those processing a polyurethane formed by reacting a mixture of polyether polyol and polyester polyol with a compound having an isocyanate (-NCO) group as a foamed foam, or those processing a polyurethane formed by reacting a hybrid copolymer of polyether polyol and polyester polyol with a compound having an isocyanate (-NCO) group as a foamed foam. In addition, in the present invention, the ether:ester ratio means the molar ratio of the ester group and the ether group in the ether/ester polyurethane foam. For example, ether:ester=4:6 means that the molar ratio of the ether group and the ester group in the polyurethane is 4:6.

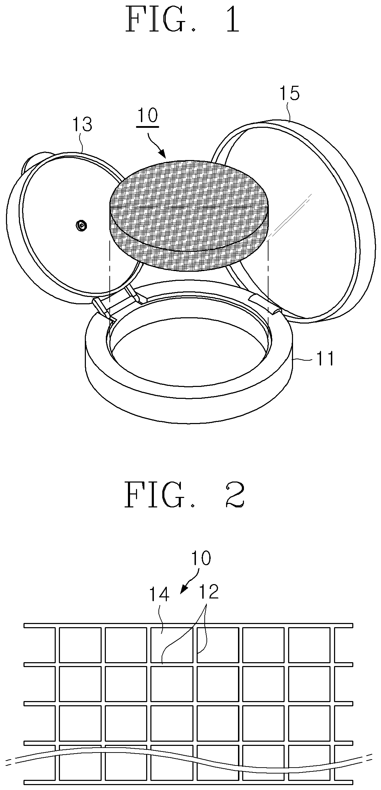

Fig. 1 is a schematic drawing of the cosmetic according to an embodiment of the present invention.

As illustrated above, the cosmetic 10 comprises a container body 11 for accommodating a liquid cosmetic substance. The container body 11 accommodates an impregnating material 100 for the liquid cosmetic substance. The cosmetic may comprise an inner lid 13 and/or an outer lid 15 for preventing leakage of the liquid cosmetic substance from the foam 100. Also, although not illustrated, in order to prevent the three-dimensional fabric impregnating material from being separated, a fixture for fixing the impregnating material may be further provided. This will be described separately later.

In the present invention, preferably, the impregnating material 100 is an ether-ester polyurethane foam. More preferably, the ether-ester polyurethane foam is an ether-ester polyurethane foam of a median ratio. Here, a median ratio means that the ratio of ester group in polyurethane is close to half. For example, preferably, the ratio of ether:ester is 4:6 to 6:4.

For example, the ether:ester ratio may be 4:6 to 6:4, preferably 4.5:5.5 to 5.5:4.5, and more preferably 4.3:5.7 to 5.7:4.3. In some embodiments of the present invention, a polyurethane foam having an ether:ester ratio of 5.5 is used. Although there is literature disclosing mixing polyether polyol and polyester polyol in polyurethane foam, no literature has been published to study the properties of polyurethane foam using polyester polyol which is conventionally known to have weak hydrolysis resistance in a ratio almost similar to polyether polyol in the raw material like the present invention, or confirm the effect thereof.

In the present invention, the discharge behavior of the cosmetic composition of an impregnating material in which a liquid cosmetic substance is impregnated may be described as follows.

The impregnating material is a porous foam, which is made up in units of cells or grids. One cell may be defined as a skeleton and a pore inside the skeleton. In the present invention, the skeleton of a cell has at least part or all of the cell wall configuring the unit lattice penetrated so that the pores of the skeleton are connected to each other. This may be referred to as an open pore structure or a reticular structure.

Fig. 2 is a two-dimensional conceptual drawing of the structure of the impregnating material 20 of the present invention. The cell comprises a skeleton 12 and a pore 14 therein defined by the skeleton. In the drawing, it is illustrated such that the skeleton 12 divides each cell. However, when viewed three-dimensionally, the skeleton 12 of a cell has an open wall and adjacent cells are in communication with each other.

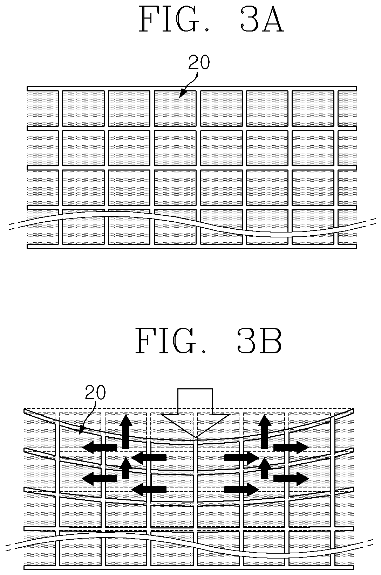

The discharge properties of the foam cosmetic in the present invention which has the cell structure of Fig. 2 will be conceptually described with reference to Figs. 3A and 3B. The following description is merely to help understand the present invention, and does not limit the technical idea of the present invention.

As illustrated in Fig. 3A, a liquid cosmetic composition 20 is impregnated in the impregnating material 10. When the surface of the impregnating material 10 is pressurized with a puff or a finger, stress is applied to the impregnating material 10 and the cosmetic composition 20. As illustrated in Fig. 3B, the unit cell in the impregnating material is defined by pressurization, and the liquid cosmetic substance therebetween flows into the cell and between adjacent cells. At this time, the skeleton of the cell acts by the resistance to the flow of the liquid cosmetic substance, whereby part or all of the pressurizing force acting vertically acts as a shear stress to the liquid cosmetic substance in the impregnating material. As such, the shear stress acting on the cosmetic substance affects the flow properties of the cosmetic composition. This will be described separately later.

With regard to the polyurethane foam configuring the impregnating material, foams using polyether polyol as raw material have excellent hydrolysis resistance, excellent elasticity, and cost less, but have weak oil resistance. In comparison, foams using polyester polyol as raw material can adjust air bubbles easily, and have good abrasion resistance, chemical resistance, etc., but are known to cause hydrolysis easily, and be broken when stored at a high temperature for a long time. In the present invention, ether-ester polyurethane foam may supplement the disadvantages of polyether foams or polyester foams of a single composition. In addition, polyester polyurethane foam can adjust the cell size more easily than polyether polyurethane foam, and may be advantageous for preparing polyurethane foam with smaller cell sizes.

In the foam of the present invention, the content (molar ratio) of the ester group in polyurethane is close to the content (molar ratio) of the ether group. Preferably, the molar ratio of ether:ester may be in a range of 6:4 to 4:6. For example, in the present invention, the molar ratio of ether:ester may be determined by the mixing ratio of polyether polyol and polyester polyol. In other words, polyurethane having an ether:ester molar ratio of 5:5 may be prepared by mixing polyether polyol and polyester polyol in a molar ratio of 5:5.

In the present invention, the cell size in the polyurethane foam may be expressed by pore number. In the present invention, the pore number may be about 70 ppi to about 250 ppi, about 80 ppi to about 250 ppi, about 90 ppi to about 250 ppi, about 100 ppi to about 250 ppi, about 110 ppi to about 250 ppi, about 120 ppi to about 250 ppi, or about 150 ppi to about 250 ppi, but is not limited thereto. Preferably, the pore number may be 110 ppi or less, 150 ppi or less, or 200 ppi or less.

The pore number is defined as the number of pores present in a 1 inch long sample of foam. Specifically, the pore number may be measured according to standards such as JIS K6400-1 as shown below.

A sample with a thickness of 10 mm, and a width and length of 100 mm is taken. The sample is placed on the pedestal of a magnifier equipped with a scale of 5 to 10 times magnification, and the magnifier or sample is moved to count the number of cells between 10 mm in a straight line. At this time, the number of cells is measured at three spots and the average of the three measurements may be calculated according to the following Equation 1 as the number of cells.

(Equation 1)

$N = n \times 2.54$ (where, N is the number of cells (pcs / 25.4 mm), and n is the number of cells between 10 mm (average))

The cell size may be expressed by the size of the pore. In the present invention, the size of the pore may be about 0.1 mm to about 0.6 mm, about 0.15 mm to about 0.6 mm, about 0.20 mm to about 0.6 mm, about 0.25 mm to about 0.60 mm, or about 0.30 mm to about 0.60 mm, but is not limited thereto. Preferably, in the present invention, the pore size is 0.5 mm or less. For example, the pore size may be 0. 1 mm to 0.5 mm, 0. 2 mm to 0.5 mm, or 0. 3 mm to 0.5 mm.

In addition, the present invention may use a foam whose degree of foam deformation occurring by pressurization is properly controlled. For example, in the present invention, the elastic modulus or the hardness of the polyurethane foam may be properly controlled. For example, the hardness of the foam may be adjusted by controlling the molecular weight of the polyol participating in the urethane reaction.

According to another aspect of the present invention, the polyurethane foam may provide good durability while having a low hardness.

In the present invention, the hardness of the polyurethane foam may be in a range of about 30 to about 60, about 35 to about 60, or about 40 to about 60 based on the ASKER durometer Type F with respect to before being impregnated with the cosmetic composition. Also, the hardness of the polyurethane foam of the present invention may be 55 or less, 50 or less, or 45 or less, but is not limited thereto.

In addition, in the present invention, the hardness of the polyurethane foam may be in a range of about 20 to about 40, about 25 to about 40, or about 30 to about 40 with respect to after being impregnated with the cosmetic composition. Also, the hardness of the polyurethane foam of the present invention may be in a range of 20 to 35.

The hardness of the polyurethane foam of the present invention is significantly lower than ordinary polyurethane foam which have a Type F ASKER hardness of about 70 to 90 before impregnation and about 40 to 45 after impregnation. However, the polyurethane foam of the present invention has properties such that the durability does not deteriorate while having a hardness lower than other polyurethane foams currently on the market. Also, as described above, the polyurethane foam of the present invention has excellent hydrolysis resistance and oil resistance.

On the other hand, in general, in case of impregnating a cosmetic composition in an impregnating material, the hardness generally decreases after impregnation. However, since the hardness does not decrease by the same value in all impregnating materials, in case there is a significant hardness difference before and after impregnation, it may be difficult to design a cosmetic composition to be impregnated in the impregnating material and this may adversely affect the sense of use of the cosmetic after impregnation. The present inventors continued researches on a polyurethane foam having excellent sense of use and effects while reducing the hardness difference before and after impregnation of the cosmetic composition as much as possible and completed the present invention.

In the present invention, the reduction rate of the hardness after impregnation compared to the hardness before impregnation of a cosmetic substance in the polyurethane foam (hardness before impregnation - hardness after impregnation / hardness before impregnation) may be less than 50%, preferably less than 45%. When the reduction rate is 50% or more, swelling of the impregnating material after impregnation may be excessively large, affecting the physical properties of the impregnating material, which may be unfavorable to the sense of use.

Further, with regard to the polyurethane foam of the present invention, it has been experimentally confirmed that the cosmetic comprising polyurethane foam as an impregnating material received a significantly higher evaluation than conventional cosmetics where polyol is included in a different ratio in terms of "sense of use" which is one of the main functional properties as can be confirmed from the following experimental experiments. It was determined that a polyurethane foam having a hardness of about 20 to about 35 after impregnation of the cosmetic composition was the softest when taking the impregnated cosmetic composition using an applicator and the amount of cosmetic composition taken by the applicator was appropriate.

In the present invention, the polyurethane foam may be prepared by reacting a mixture of polyether polyol and polyester polyol with a compound having an isocyanate (-NCO) group.

The monomer of the polyether polyol may be selected from a group consisting of alkylene oxide, diol, triol, or combinations thereof. The monomer of the polyester polyol may comprise an organic acid comprising two or more carboxyl groups, but is not limited thereto. In an embodiment of the present invention, the monomer of the polyether polyol may comprise an alkylene oxide selected from a group consisting of ethylene oxide and propylene oxide; diol selected from a group consisting of dipropylene glycol (hereinafter also referred to as "DPG"), monoethylene glycol (hereinafter referred to as "MEG"), and propylene glycol (hereinafter referred to as "PG"); or a triol selected from a group consisting of glycerin and trimethylol propane (hereinafter also referred to as "TMP"); or combinations thereof, but is not limited thereto. In an embodiment of the present invention, the polyether polyol may be obtained by addition polymerization of propylene oxide or ethylene oxide, which is an alkylene oxide, to an initiator having activated hydrogen, but is not limited thereto. As the initiator, propylene glycol, dipropylene glycol, ethylene glycol, diethylene glycol, diethanol amine, bisphenol, water, etc. which have two functional groups, or glycerine, trimethylol propane, triethanol amine, haxanetriol, etc. which have three functional groups are mainly used, but the initiator is not limited thereto.

In the present invention, the monomer of the polyester polyol comprises a polyvalent organic acid having about 2 to about 20 carbon atoms comprising two or more carboxyl groups. For example, the monomer of the polyester polyol may comprise higher fatty acid, saturated fatty acid, or unsaturated fatty acid, but is not limited thereto. For example, the number of carbon atoms of the polyvalent organic acid may be about 2 to about 20, about 2 to about 18, about 2 to about 16, about 2 to about 14, about 2 to about 12, about 2 to about 10, about 2 to about 8, about 2 to about 6, or about 2 to about 4. In an embodiment of the present invention, the polyvalent organic acid comprising two or more carboxyl groups may be selected from a group consisting of, for example, adipic acid, oxalic acid, and succinic acid, but is not limited thereto. In an embodiment of the present invention, the monomer of the polyester polyol may be selected from a group consisting of soy bean oil, castor oil, citric oil, palmitic acid, myristic acid, oleic acid, stearic acid, linoleic acid and linolenic acid, but is not limited thereto.

In the present invention, the compound having an isocyanate (-NCO) group may be selected from a group consisting of polyisocyanate having two or more isocyanate groups such as aromatic, alicyclic or aliphatic polyisocyanate, combinations of two or more of the polyisocyanates, and modified polyisocyanates obtained by modifying the above, but is not limited thereto. In an embodiment of the present invention, the compound having an isocyanate (-NCO) group may be methylene diphenyl diisocyanate (hereinafter referred to as "MDI"), toluene diisocyanate (hereinafter referred to as "TDI"), aromatic diisocyanates such as 2,4- or 2,6-tolylene diisocyanate, xylylene diisocyanate (XDI), paraphenylene diisocyanate, 1,5-naphthalene diisocyanate, and tridine diisocyanate; aliphatic diisocyanates having aromatic rings such as $\alpha$, $\alpha$, $\alpha$', $\alpha$'-tetramethyl xylylene diisocyanate; aliphatic diisocyanates such as methylene diisocyanate, propylene diisocyanate, lysine diisocyanate, 2,2,4- or 2,4,4-trimethylhexamethylene diisocyanate and 1,6-hexamethylene diisocyanate (HMDI); alicyclic diisocyanates such as 1,4-cyclohexane diisocyanate, methylcyclohexane diisocyanate (hydrogenated TDI), 1-isocyanate-3-isocyanatemethyl-3,5,5-trimethylcyclohexane (IPDI), 4,4'-dicyclohexylmethaned iisocyanate, isopropylidenedicyclohexyl-4,4'-diisoate, or combinations thereof, but is not limited thereto.

Hereinafter, the properties of a cosmetic composition suitable to be impregnated in the ether-ester polyurethane foam of the present invention described above will be explained.

In order to supplement the hydrolysis properties of the polyurethane foam having a high ester content described above, the cosmetic composition of the present invention has a low water content. Preferably, the water content of the cosmetic composition is 35 wt% or less, and more preferably 30 wt% or less. For example, the water content of the cosmetic composition may be about 20 to 35 wt%, 20 to 30 wt%, or 25 to 30 wt% of the cosmetic composition.

In addition, the cosmetic composition in the present invention has the following flow properties.

The flow properties of the cosmetic substance of the present invention may be expressed by storage modulus (G') and loss modulus (G") which represent the elasticity and the viscosity of the composition.

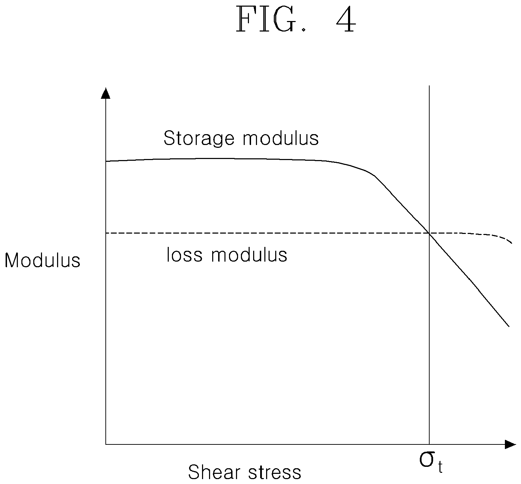

Fig. 4 is a schematic drawing for illustrating the flow of the cosmetic composition according to an embodiment of the present invention. Referring to Fig. 4, when no shear stress is applied, the storage modulus of the composition is greater than the loss modulus (G"). Storage modulus is an index representing the elastic properties of a substance. When no shear stress is applied, the composition presents a behavior close to that of a solid, i.e., a solid-like behavior. However, when shear stress is applied and the stress increases, the storage modulus gradually decreases and eventually the storage modulus and loss modulus are reversed. At this time, the shear stress at a point where G'=G" is expressed as threshold shear stress ($\sigma_t$). When the shear stress exceeds a threshold, the composition is superior in viscosity properties, i.e., the composition presents a state of G">G', which is a liquid-like behavior. At this time, the composition of a liquid-like behavior has flow properties close to those of liquid and may easily flow between cells of the impregnating material.

The cosmetic composition of the present invention has low threshold shear stress. For example, the threshold shear stress ($\sigma_t$) may be about 100 Pa or less, about 90 Pa or less, about 80 Pa or less, about 70 Pa or less, about 60 Pa or less, about 50 Pa or less, about 40 Pa or less, about 30 Pa or less, about 20 Pa or less, about 10 Pa or less, about 9 Pa or less, about 8 Pa or less, about 7 Pa or less, about 6 Pa or less, about 5 Pa or less, 4 Pa or less, 3 Pa or less, 2 Pa or less, or 1 Pa or less. Preferably, the threshold shear stress of the composition may be in a range of 1 Pa to 20 Pa, 2 to 20 Pa, 3 to 20 Pa. The above-described storage modulus and loss modulus may be measured by a commonly used rheometer.

As described above, the cosmetic composition in the pressurized polyurethane foam undergoes shear stress. Due to shear stress, the composition of the present invention behaves like a liquid, and easily flows inside the foam. Such flow properties may provide advantages in discharging the cosmetic uniformly. For example, when repeatedly used several times, the composition of the present invention may quickly flow into the impregnating material and be rearranged. When used, the composition in the impregnating material is continuously rearranged, enabling more uniform discharge during the use period. Also, since the flow properties of the composition may improve by pressurization, the cosmetic composition may be easily impregnated by repeatedly pressurizing the foam during filling.

In comparison, the composition of the present invention behaves like a solid in a state where no shear stress is applied, and accordingly may exist as a more stable composition in the foam. In order to secure stability in a non-stressed state, preferably the cosmetic composition has a relation of G'>>G", more specifically a relation of $3 \leq G'G" \leq 10$ under a shear stress of 1 Pa.

The cosmetic composition of the present invention comprises an oil phase component, an aqueous phase component, a thickener and powder. These components and the contents thereof determine the flow properties of the cosmetic composition.

The composition of the present invention may comprise an oil phase component of 38~43 wt%, an aqueous phase component of 38~43 wt%, a pigment of 15~20 wt%, and a thickener of less than 1 wt% with regard to the entire cosmetic composition.

In the present invention, the oil phase component may include an oil, a film-forming agent, a sunscreen and an emulsifying agent.

In the present invention, the emulsifying agent may comprise a main emulsifying agent and an auxiliary emulsifying agent. In the present invention, the emulsifying agent may be used in an amount of 8 to 12 parts by weight with respect to 100 parts by weight of the oil phase component.

In the present invention, the main emulsifying agent comprises at least one substance selected from a group consisting of lauryl PEG-10 tris (trimethylsiloxy) silylethyl dimethicone, lauryl PEG-9 polydimethyl siloxyethyl dimethicone and cetyl PEG/PPG-10/1 dimethicone.

In the present invention, at least one of PEG-10 dimethicone and sorbitan isostearate may be used as the auxiliary emulsifying agent.

In the present invention, preferably, the main emulsifying agent and the auxiliary emulsifying agent may be used in a ratio of 3:1 to 4:1. When the ratio of the main emulsifying agent and the auxiliary emulsifying agent is less than 3:1, long-term formulation stability may drop.

In the present invention, preferably, the weight ratio of the oil phase component to the aqueous phase component may be in a range of 0.9 to 1.1, more preferably 0.95 to 1.1. When the ratio of the aqueous phase component is lower, the emulsification stability may drop sharply.

**Mode for carrying out the invention**

Hereinafter, preferred embodiments of the present invention will be described.

A. Polyurethane foam

A composite polyurethane foam prepared by varying the ratio of polyester polyol and polyether polyol is prepared. For comparison, a polyester polyol 100% polyurethane foam and a polyether polyol 100% polyurethane foam are prepared.

The composition of each polyurethane foam is shown in Table 1 below.

[Table 1]

|      | Ether : ester ratio |
|------|---------------------|
| PU1  | 7 : 3               |
| PU2  | 6 : 4               |
| PU3  | 5 : 5               |
| PU4  | 4 : 6               |
| PU5  | 3 : 7               |
| PU6  | 10 : 0              |
| PU7  | 0 : 10              |

In order to evaluate the hydrolysis properties of the foamed foam, the polyester polyol foam and the hybrid foamed foam are impregnated in 10% hydrochloric acid and 10% nitric acid for 7 days at room temperature and whether the foam has collapsed is checked. In order to proceed the experiment under accelerated conditions, the evaluation is carried out in an aqueous solution of 10% hydrochloric acid and 10% nitric acid, which are acid catalysts. The experiment results are shown in Table 2 below.

[Table 2]

|     | 10% hydrochloric acid | 10% nitric acid |
|-----|-----------------------|-----------------|
| PU2 | ○                     | ○               |
| PU3 | ○                     | ○               |
| PU4 | ○                     | ○               |

(continued)

|  | 10% hydrochloric acid | 10% nitric acid |
|---|---|---|
| PU5 | ○ | ○ |
| PU6 | ○ | ○ |
| PU7 | X | X |

(O: does not collapse, X: collapses) Ester oil, polar organic UV blocking agent and volatile silicone oil included in the components of the cosmetic composition are impregnated respectively, and maintained for 20 minutes at 80°C. Then, the size of each urethane foam is measured and compared to the volume of the foamed foam before impregnation to confirm the volume expansion degree of the foamed foam. The results are shown in Table 3 below. As an ester oil, cetylethylhexanoate, diisostearyl malate, butylene glycol dicaprylate/dicaprate, dicaprylyl carbonate, and neopentyl glycol diheptanoate are used. As a polar organic UV blocking agent, ethylhexyl methoxycinnamate, hexyl salicylate, and isoamyl-P-methoxycinnamate are used. As a volatile silicone oil, cyclohexasiloxane, sariclopentasiloxane, and cyclomethicone are used.

[Table 3]

|  | Ester oil | Polar organic UV blocking agent | Volatile silicone oil |
|---|---|---|---|
| PU2 | 2.2% | 6.0% | 0% |
| PU3 | 1.8% | 5.1% | 0% |
| PU4 | 1.5% | 4.8% | 0% |
| PU5 | 1.2% | 4.6% | 0% |
| PU6 | 4.5% | 8.0% | 0% |
| PU7 | 1.0% | 4.5% | 0% |

B. Cosmetic composition

A W/O type cosmetic composition is prepared. Oil phase components including sunscreen, film-forming agent, emulsifying agent and oil are mixed, heated and stirred at 80°C to make the phase uniform. Powder is mixed thereto and stirred again to prepare an oil phase component in which the powder components are uniformly dispersed. An aqueous phase component is prepared by mixing aqueous phase components in a separate beaker and heating and stirring the mixture at 80°C to dissolve the mixture completely. The prepared aqueous phase components are slowly introduced into the oil phase components prepared as above and emulsified using a homo mixer. Then, the mixture is cooled to 30°C to obtain the cosmetic compositions in Tables 5 to 7.

The cosmetic composition is designed in a composition having low threshold shear stress at a water content of 30 wt% or less. The component and combination ratio (wt%) of the prepared cosmetic composition are as shown in Table 4 below.

[Table 4]

|  |  | Preparation example 1 | Preparation example 2 | Preparation example 3 |
|---|---|---|---|---|
| Component | Oil phase component | 39.7 | 42.5 | 41.2 |
|  | Aqueous phase component (water content) | 41.9(26.4) | 39.3(29.8) | 38.8(26.8) |
|  | Thickener | 0.6 | 0.7 | 0.6 |
|  | Powder | 17.8 | 17,5 | 18.2 |

The specific combination ratio of preparations 1 to 3 is as shown in Tables 5 to 7.

[Table 5]

| | | Name | Content |
|---|---|---|---|
| Oil phase | Oil | Cyclopentasiloxane | 17.25 |
| | | Dicaprylyl carbonate | 5.00 |
| | | Phenyl trimethicone | 4.00 |
| | | Butylene glycol dicaprylate/dicaprate | 3.00 |
| | | Isododecane | 1.20 |
| | Film-forming agent | Polypropylsilsesquioxane | 2.25 |
| | | Trimethylsiloxysilicate | 1.80 |
| | Emulsifying agent | Lauryl PEG-10 tris (trimethylsiloxy) silylethyl dimethicone | 3.00 |
| | | PEG-10 dimethicone | 1.00 |
| Thickener | | Disteadimonium hectorite | 0.60 |
| Aqueous phase | | Purified water | 26.44 |
| | | Butylene glycol | 10.00 |
| | | Niacinamide | 2.00 |
| | | Pentylene glycol | 2.00 |
| | | Magnesium sulfate | 0.70 |
| | | Phenoxy ethanol | 0.63 |
| | | Ethylhexyl gylcerin | 0.07 |
| | | Adenosine | 0.04 |
| | | Disodium EDTA | 0.02 |
| Powder | | Pigment | 17.76 |
| | | Methyl methacrylate cross polymer | 1.24 |

[Table 6]

| | | Name | Content |
|---|---|---|---|
| Oil phase | Oil | Cyclopentasiloxane | 8.65 |
| | | Phenyl trimethicone | 5.00 |
| | | Cyclohexasiloxane | 3.50 |
| | | Butylene glycol dicaprylate/dicaprate | 3.00 |
| | | Tridecyl trimellitate | 3.00 |
| | | Dimethicone/vinyl dimethicone cross polymer | 0.35 |
| | Sunscreen | Ethylhexyl methoxy cinnamate | 7.00 |
| | | Ethylhexyl salicylate | 4.50 |
| | Film-forming agent | Trimethylsiloxysilicate | 3.00 |
| | | Polypropylsilsesquioxane | 0.50 |
| | Emulsifying agent | Lauryl PEG-10 tris (trimethylsiloxy) silylethyl dimethicone | 3.00 |
| | | Cetyl PEG/PPG-10/1 dimethicone | 1.00 |
| Thickener | | Disteadimonium hectorite | 0.70 |
| Aqueous phase | | Purified water | 29.84 |

(continued)

| | Name | Content |
|---|---|---|
| | Glycerin | 3.00 |
| | Niacinamide | 2.00 |
| | Pentylene glycol | 2.00 |
| | Butylehne glycol | 1.00 |
| | Magnesium sulfate | 0.70 |
| | Phenoxy ethanol | 0.63 |
| | Ethylhexyl gylcerin | 0.07 |
| | Adenosine | 0.04 |
| | Disodium EDTA | 0.02 |
| Powder | Pigment | 16.65 |
| | Methyl methacrylate cross polymer | 0.85 |

[Table 7]

| | | Name | Content |
|---|---|---|---|
| Oil phase | Oil | Methyl trimethicone | 8.00 |
| | | Dimethicone | 7.74 |
| | | Butylene glycol dicaprylate/dicaprate | 3.50 |
| | | Diphenyl siloxy phenyl trimethicone | 3.00 |
| | | Trisiloxane | 1.50 |
| | | Dimethicone cross polymer | 0.80 |
| | | Phenyl trimethicone | 0.50 |
| | Sunscreen | Ethylhexyl methoxy cinnamate | 6.70 |
| | | Ethylhexyl salicylate | 4.00 |
| | Film-forming agent | Acrylate/stearyl acrylate/dimethicone methacrylate copolymer | 1.00 |
| | Emulsifying agent | Lauryl PEG-10 tris (trimethylsiloxy) silylethyl dimethicone | 3.50 |
| | | PEG-10 dimethicone | 1.00 |
| Thickener | | Disteadimonium hectorite | 0.60 |
| Aqueous phase | | Purified water | 28.05 |
| | | Glycerin | 4.00 |
| | | Butylene glycol | 3.60 |
| | | Niacinamide | 2.00 |
| | | 1,2-hexanediol | 1.50 |
| | | Magnesium sulfate | 0.70 |
| | | Glyceryl caprylate | 0.10 |
| | | Adenosine | 0.04 |
| | | Disodium EDTA | 0.02 |

(continued)

|  | Name | Content |
|---|---|---|
| Powder | Pigment | 17.14 |
|  | Mica | 1.01 |

The viscosity and flow properties of the cosmetic compositions in preparation examples 1 to 3 are measured. The viscosity is measured by a viscometer (DV-E Viscometer, Brookfield) after putting 100 g of each of the prepared composition in a plastic container. At this time, Spindle No. 4 is used when measuring the viscosity, and the value obtained by dipping the spindle in each composition and rotating at 12 rpm for 1 minute is used as the viscosity. The result of measuring the viscosity is shown in Table 8 below.

[Table 8]

|  | Preparation example 1 | Preparation example 2 | Preparation example 3 |
|---|---|---|---|
| Viscosity (cps) | 6,050 | 9,450 | 11,050 |

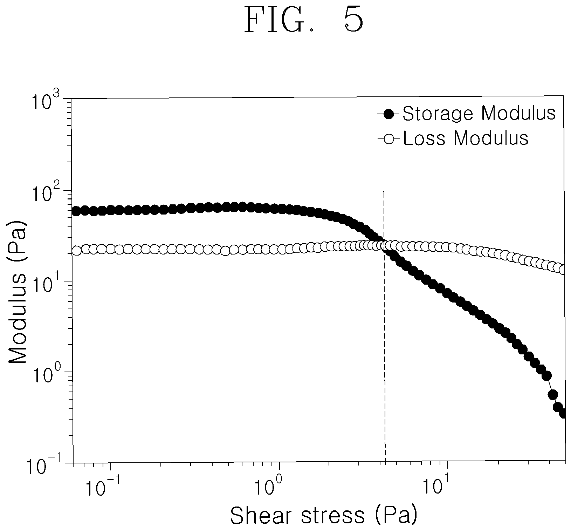

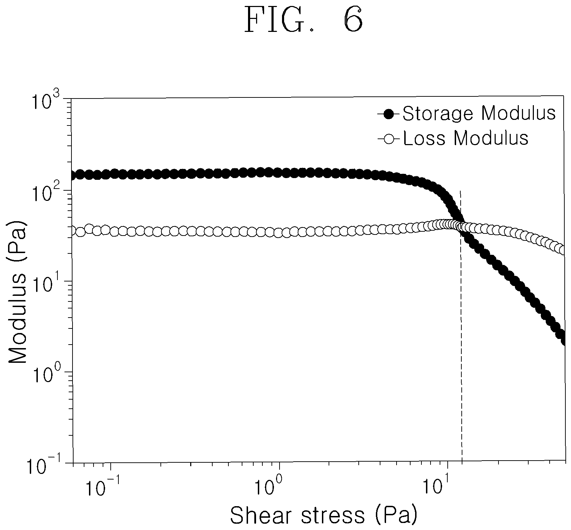

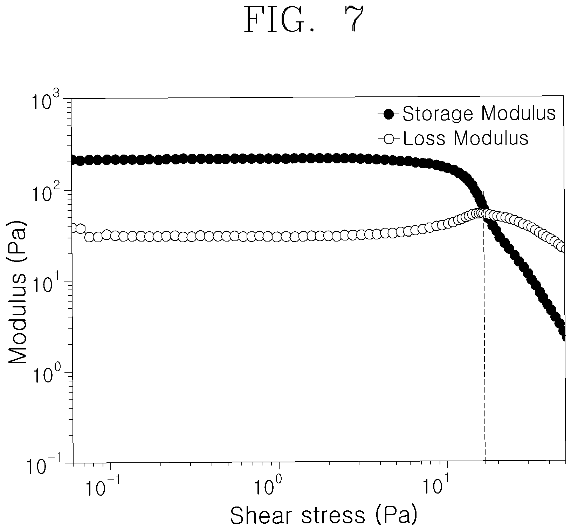

The flow properties of the compositions in preparations 1 to 3 are measured by a cone- and-plate rheometer (MCR92, Anton Paar). At this time, the measurement frequency is 10 Hz. Figs. 5 to 7 are graphs illustrating the storage modulus and the loss modulus as measured, and Table 9 summarizes the threshold shear stress of Figs. 5 to 7.

[Table 9]

|  | Preparation example 1 | Preparation example 2 | Preparation example 3 |
|---|---|---|---|
| Threshold shear stress ($\sigma_t$) | about 3.1 Pa | about 10.1 Pa | about 15 Pa |

C. Evaluation of rearrangement properties of cosmetic composition

Impregnation foams are prepared by impregnating each composition in preparations 1 to 3 into PU3 foam which has a thickness of about 10 mm and a diameter of about 5 cm. The weight of the composition impregnated in each foam is 16 g. The impregnated PU3 foam is used as a lower PU3 foam, and an upper PU3 foam not impregnated is placed thereon and pressurized so that the total thickness of the PU3 foam is 15 cm and maintained for 10 seconds, and then the relative size of the weight reduction of the lower PU3 foam is compared. As a result of measuring the weight, the weight loss is greater in the order of preparation example 1, preparation example 2 and preparation example 3. This seems to be because the lower the threshold shear stress of the impregnated composition, the more actively the composition flows to the upper PU3 foam, and the greater the weight loss is in the composition of the lower PU3 foam.

From the above experiment, it can be confirmed that a significant amount of weight loss occurred in the lower foam, so that the composition can be rearranged easily during pressurization. Also, it can be understood that a relatively large amount of weight loss occurred in the case of a foam supporting a composition having low threshold shear stress.

D. Hardness evaluation before and after impregnation

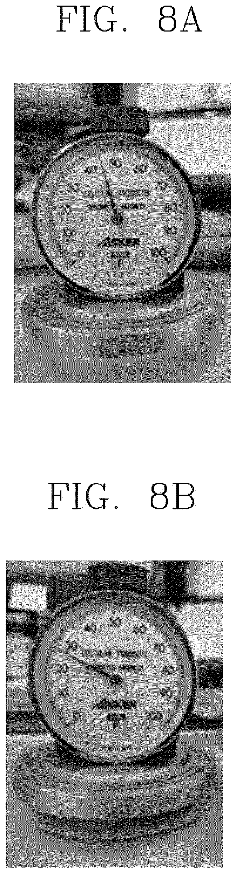

The hardness is measured before and after impregnation of PU1, PU3, PU6 foams which have a thickness of about 10 mm and a diameter of about 5 cm. The impregnation composition is prepared in a composition of preparation example 2, and the impregnation amount was 16 g. For comparison, the hardness before and after impregnation of PU4 foam is measured. The hardness is calculated based on the ASKER durometer Type F. Figs. 8A and 8B are photographs of the hardness measuring process of the PU3 foam before and after impregnation, respectively.

The results of the hardness measurement and hardness change rate of the PU2 foam used in the experiment are shown in Table 10 below.

[Table 10]

|  | Hardness before impregnation | Hardness after impregnation | Hardness change rate (%) |
|---|---|---|---|
| PU1 | 87 | 45 | 48 |
| PU3 | 45 | 26 | 42 |

(continued)

|  | Hardness before impregnation | Hardness after impregnation | Hardness change rate (%) |
|---|---|---|---|
| PU6 | 75 | 38 | 49 |

E. Sensory evaluation

After impregnating the cosmetic composition of preparation example 1 in PU1, PU3, PU6 polyurethane foams, a sensory evaluation is carried out on 20 women in their 20s and 30s who are talented in evaluating cosmetics in order to confirm the difference in effect according to the hardness difference before and after impregnating the cosmetic substance. In the present experiment, PU1, PU3 and PU6 foams are used as polyurethane foam whose hardness is to be evaluated. All three types of polyurethane foams are identical to the naked eye, and sensory evaluation is carried out without providing any background description on the polyurethane foam to the subjects.

Three types of sensory properties including sense of discharge, sense of contact and sense of use are evaluated. The sensory properties are evaluated based on a 5-point scale where 5 indicates very good, 4 indicates good, 3 indicates average, 2 indicates poor, and 1 indicates very poor, and the average is calculated. Explanation on each item in the sensory evaluation is as shown below.

1) Sense of discharge: Whether the amount discharged when taking the cosmetic composition from the impregnating material with an applicator (ordinary NBR puff) is appropriate is evaluated. This may affect the durability of the impregnating material because the cosmetic composition needs to be taken several times when the one-time discharge amount is too small.

2) Sense of contact: Sensory properties felt when the applicator is in contact with the impregnating material are evaluated.

3) Sense of use: Overall sense of use felt when taking the impregnated cosmetic composition with an applicator is evaluated.

[Table 11]

|  | Example | Comparative Example 1 | Comparative Example 2 |
|---|---|---|---|
| Sense of discharge | 4.6 | 3.9 | 4.2 |
| Sense of contact | 4.2 | 4.1 | 3.8 |
| Sense of use | 4.3 | 3.8 | 3.6 |
| Average | 4.4 | 3.9 | 3.8 |

As shown in Table 11 above, the sensory properties of the polyurethane foam of the example according to the present invention are evaluated to be superior to those of comparative examples 1 and 2. It is to be noted that the sense of discharge in the example with a low reduction rate before and after impregnation received a significantly higher evaluation than the comparative example with a high reduction rate, and the example showed an excellent effect in terms of the overall sense of use.

As described above, the present invention has been described through the embodiments of the present invention, but the above description is only illustrative of the present invention and the present invention is not limited thereto. Any person having ordinary knowledge in the field to which the present invention pertains should regard the scope of the present invention to extend to a range where various modifications or changes are made without departing from the scope of the appended claims and the present invention.

**Industrial applicability**

The present invention may be used for cosmetics.

**Claims**

1. A cosmetic comprising polyurethane foam in which a cosmetic composition is impregnated, wherein the polyurethane of the polyurethane foam comprises an ether group and an ester group in a molar ratio of 4:6 to 6:4, the cosmetic composition has a water content of 35 wt% or less, and the threshold shear stress at which storage modulus (G')=loss modulus (G") is 100 Pa or less.

2. The cosmetic of claim 1, wherein the water content of the cosmetic composition is 20 to 35 wt%.

3. The cosmetic of claim 1, wherein the water content of the cosmetic composition is 20 to 30 wt%.

4. The cosmetic of claim 1, wherein the water content of the cosmetic composition is 25 to 30 wt%.

5. The cosmetic of claim 1, wherein the threshold shear stress of the cosmetic composition is 20 Pa or less.

6. The cosmetic of claim 1, wherein the threshold shear stress of the cosmetic composition is 10 Pa or less.

7. The cosmetic of claim 1, wherein the threshold shear stress of the cosmetic composition is 5 Pa or less.

8. The cosmetic of claim 1, wherein the cosmetic composition is $3 \leq G'G" \leq 10$ under a shear stress of 1 Pa.

9. The cosmetic of claim 1, wherein the cosmetic composition comprises an oil phase component and an aqueous phase component, and the weight ratio of the oil phase component to the aqueous phase component is in a range of 0.9 to 1.1.

10. The cosmetic of claim 1, wherein the cosmetic composition comprises a main emulsifying agent and an auxiliary emulsifying agent, and the weight ratio of the main emulsifying agent and the auxiliary emulsifying agent is in a range of 3:1 to 4:1.

11. The cosmetic of claim 1, wherein the main emulsifying agent comprises at least one substance selected from the group consisting of lauryl PEG-10 tris (trimethylsiloxy) silylethyl dimethicone, lauryl PEG-9 polydimethyl siloxyethyl dimethicone and cetyl PEG/PPG-10/1 dimethicone.

12. The cosmetic of claim 1, wherein the auxiliary emulsifying agent comprises at least one of PEG-10 dimethicone and sorbitan isostearate.

13. The cosmetic of claim 1, wherein the hardness after impregnating the polyurethane foam is 20 to 35.

14. The cosmetic of claim 1, wherein the hardness change rate before and after impregnation with respect to the hardness of the polyurethane foam before impregnation is 45% or less.

# FIG. 1

# FIG. 2

# FIG. 3A

# FIG. 3B

# FIG. 4

Storage modulus

Modulus

loss modulus

Shear stress

$\sigma_t$

# FIG. 5

FIG. 6

# FIG. 7

# FIG. 8A

# FIG. 8B

## INTERNATIONAL SEARCH REPORT

| | International application No. |
|---|---|
| | **PCT/KR2018/014364** |

**A. CLASSIFICATION OF SUBJECT MATTER**

*A61K 8/02(2006.01)i, A61K 8/891(2006.01)i, A61Q 1/02(2006.01)i, C08G 18/40(2006.01)i*

According to International Patent Classification (IPC) or to both national classification and IPC

**B. FIELDS SEARCHED**

Minimum documentation searched (classification system followed by classification symbols)
A61K 8/02; A45D 34/00; A45D 40/00; A61K 8/04; A61K 8/06; A61K 8/72; A61K 8/87; A61K 8/891; A61Q 1/02; C08G 18/40

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: polyurethane foam, impregnated material, cosmetic composition, polyether polyol, polyester polyol, water content, threshold shear stress, emulsifier

**C. DOCUMENTS CONSIDERED TO BE RELEVANT**

| Category* | Citation of document, with indication, where appropriate, of the relevant passages | Relevant to claim No. |
|---|---|---|
| X | KR 10-2016-0106000 A (SEO, Eungyeong) 09 September 2016<br>See claims 1-4; and paragraphs [0024], [0028], [0042]. | 1-14 |
| A | WO 2017-209484 A1 (COSMAX, INC.) 07 December 2017<br>See claim 1; and paragraphs [59], [111]-[112], [116], [129]. | 1-14 |
| A | KR 10-2018-0064888 A (COREANA COSMETICS CO., LTD.) 15 June 2018<br>See claim 1. | 1-14 |
| A | KR 10-2012-0108509 A (AMOREPACIFIC CORPORATION) 05 October 2012<br>See the entire document. | 1-14 |
| A | KR 10-2014-0094485 A (AMOREPACIFIC CORPORATION) 30 July 2014<br>See the entire document. | 1-14 |

☐ Further documents are listed in the continuation of Box C.   ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 17 JULY 2019 (17.07.2019) | **18 JULY 2019 (18.07.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2018/014364**

| Patent document cited in search report | Publication date | Patent family member | Publication date |
|---|---|---|---|
| KR 10-2016-0106000 A | 09/09/2016 | KR 10-1679320 B1 | 24/11/2016 |
| WO 2017-209484 A1 | 07/12/2017 | CN 108601435 A | 28/09/2018 |
| | | EP 3466298 A1 | 10/04/2019 |
| | | JP 10-2019-504681 A | 21/02/2019 |
| | | KR 10-1756071 B1 | 11/07/2017 |
| | | US 2019-0029394 A1 | 31/01/2019 |
| KR 10-2018-0064888 A | 15/06/2018 | KR 10-1898244 B1 | 13/09/2018 |
| KR 10-2012-0108509 A | 05/10/2012 | CA 2804298 A1 | 27/09/2012 |
| | | CA 2804298 C | 03/04/2018 |
| | | CA 2998181 A1 | 27/09/2012 |
| | | CN 103221029 A | 24/07/2013 |
| | | CN 103221029 B | 16/11/2016 |
| | | CN 106890107 A | 27/06/2017 |
| | | CN 107028803 A | 11/08/2017 |
| | | CN 107412008 A | 01/12/2017 |
| | | EP 2574166 A2 | 03/04/2013 |
| | | EP 2574166 A4 | 19/02/2014 |
| | | EP 2574166 B1 | 18/10/2017 |
| | | EP 3295930 A1 | 21/03/2018 |
| | | EP 3295930 B1 | 21/11/2018 |
| | | ES 2647449 T3 | 21/12/2017 |
| | | JP 2013-530252 A | 25/07/2013 |
| | | JP 2014-129351 A | 10/07/2014 |
| | | JP 5465357 B2 | 09/04/2014 |
| | | JP 6254850 B2 | 27/12/2017 |
| | | KR 10-1257628 B1 | 29/04/2013 |
| | | MY 154938 A | 18/08/2015 |
| | | SG 186767 A1 | 28/02/2013 |
| | | TW 201238990 A | 01/10/2012 |
| | | TW I516516 B | 11/01/2016 |
| | | US 2014-0023689 A1 | 23/01/2014 |
| | | US 2019-0117524 A1 | 25/04/2019 |
| | | WO 2012-128589 A2 | 27/09/2012 |
| | | WO 2012-128589 A3 | 27/12/2012 |
| KR 10-2014-0094485 A | 30/07/2014 | CN 104379124 A | 25/02/2015 |
| | | CN 104379124 B | 13/06/2017 |
| | | EP 2837375 A1 | 18/02/2015 |
| | | EP 2837375 A4 | 02/12/2015 |
| | | EP 2837375 B1 | 10/01/2018 |
| | | HK 1203381 A1 | 30/10/2015 |
| | | JP 2015-512934 A | 30/04/2015 |
| | | JP 6355620 B2 | 11/07/2018 |
| | | KR 10-2013-0116044 A | 22/10/2013 |
| | | MY 166285 A | 25/06/2018 |
| | | PH 12014502300 A1 | 22/12/2014 |

Form PCT/ISA/210 (patent family annex) (January 2015)

**INTERNATIONAL SEARCH REPORT**
Information on patent family members

International application No.

**PCT/KR2018/014364**

| Patent document cited in search report | Publication date | Patent family member | Publication date |
|---|---|---|---|
| | | PH 12014502300 B1 | 22/12/2014 |
| | | SG 11201406400Q A | 27/11/2014 |
| | | TW 201345557 A | 16/11/2013 |
| | | TW I580437 B | 01/05/2017 |
| | | US 2015-0104235 A1 | 16/04/2015 |
| | | US 9532637 B2 | 03/01/2017 |
| | | WO 2013-154395 A1 | 17/10/2013 |

Form PCT/ISA/210 (patent family annex) (January 2015)